(19) Europäisches Patentamt
European Patent Office
Office européen des brevets

(11) Numéro de publication: **0 020 765 A1**

(12) # DEMANDE DE BREVET EUROPEEN

publiée en application de l'article 158, paragraphe 3 de la CBE

(21) Numéro de dépôt: **79900778.6**

(51) Int. Cl.³: **A 61 K 31/47**

(22) Date de dépôt: **13.07.79**

Données relatives à la demande internationale prise pour base:
(86) Numéro de dépôt international: **PCT/JP 79/00185**
(87) Numéro de publication internationale: **WO 80/00215 (21.02.80 80/4)**

(30) Priorité: **14.07.78 JP 86231/78**

(43) Date de publication de la demande: **07.01.81 Bulletin 81/1**

(84) Etats contractants désignés: **FR**

(71) Demandeur: **OTSUKA PHARMACEUTICAL CO., LTD., 9, Kandatsukasacho 2-chome, Chiyoda-ku, Tokyo 101 (JP)**

(72) Inventeur: **YABUUCHI, Yoichi, 692-2, Omatsu, Kawauchicho, Tokushima-shi Tokushima 771-01 (JP)**
Inventeur: **YOSHIZAKI, Shiro, 2-44, Aya Sotobiraki Tarohachisu, Kitajima-cho, Itano-gun Tokushima 771-02 (JP)**
Inventeur: **NAKAGAWA, Kazuyuki, 774, Omatsu Kawauchicho, Tokushima-shi Tokushima 771-01 (JP)**

(74) Mandataire: **Ores, Irène et al, CABINET ORES 6, Avenue de Messine, F-75008 - Paris (FR)**

(54) **REMEDE CONTRE LE GLAUCOME.**

(57) Un remède contre le glaucome contenant comme composant effectif un composé carbostyrile ayant une action de suppression de la pression intra-oculaire et étant représenté par la formule générale suivante:

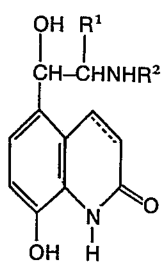

(I)

où $R^1$ et $R^2$ représentent chacun un groupe alkyle inférieur, et la liaison carbone-carbone entre les positions 3 et 4 de la structure carbostyrile est une liaison simple ou double.

EP 0 020 765 A1

TITRE MODIFIÉ
voir page de garde

COMPOSITIONS POUR LE TRAITEMENT DU GLAUCOME

La présente invention est relative à des compositions pour le traitement du glaucome.

Le glaucome est fondamentalement imputable à une augmentation soutenue ou répétée de la pression intraoculaire, et il est à l'origine des troubles fonctionnels, ainsi que des troubles organiques de l'oeil. Pour traiter cette maladie, on considère qu'il faut abaisser rapidement la pression intraoculaire anormalement élevée jusqu'à sa valeur normale, pour conserver une acuité visuelle convenable (Masakichi Mikuni et Kazuo Iwata, "Glaucome", Kanehara Shuppan Co., Ltd., 1968).

Les compositions pour le traitement du glaucome conformes à la présente invention comprennent en tant que constituant actif, un dérivé de carbostyrile représenté par la formule I ci-après :

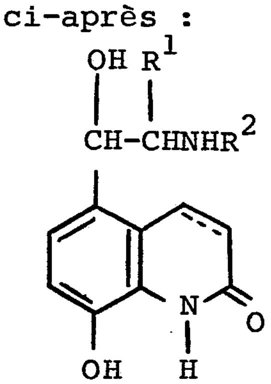

(I)

dans laquelle :

$R^1$ et $R^2$ représentent chacun un groupe alkyle inférieur, et

la liaison carbone-carbone entre les positions 3 et 4 du squelette carbostyrilique est une liaison simple ou double,

ou un sel d'addition avec un acide d'un tel dérivé.

Les groupes alkyle inférieur représentés par $R^1$ et $R^2$ dans la formule (I), sont des groupes alkyle à chaîne droite ou ramifiée comportant de 1 à 4 atomes de carbone, comme les radicaux méthyle, éthyle, propyle, isopropyle, butyle, tert-butyle, etc... Parmi les sels d'addition avec un acide des dérivés de carbostyrile représentés par la formule (I), actifs, on compte des sels acceptables du point

de vue pharmaceutique, tels que, par exemple, des sels de l'acide chlorhydrique, de l'acide sulfurique, de l'acide nitrique, de l'acide bromhydrique, de l'acide oxalique, de l'acide maléique, de l'acide fumarique, de l'acide citrique, de l'acide tartrique, etc...

On trouvera ci-après, quelques exemples caractéristiques de composés actifs de formule (I) utiles dans les compositions de traitement du glaucome conformes à la présente invention :

- 8-hydroxy-5-(1-hydroxy-2-isopropylaminobutyl)carbostyrile
- 8-hydroxy-5-(1-hydroxy-2-tert-butylaminopropyl)-carbostyrile
- 8-hydroxy-5-(1-hydroxy-2-éthylaminobutyl)-3,4-dihydrocarbostyrile
- 8-hydroxy-5-(1-hydroxy-2-isopropylaminobutyl)-3,4-dihydrocarbostyrile.

Les composés représentés par la formule (I) utiles en tant que constituants actifs, sont des composés connus préparés par exemple en mettant en oeuvre le procédé décrit dans la Demande de Brevet japonais publiée après examen n° 10994/1978. Ces composés actifs exercent une activité bronchodilatatrice et sont utiles dans le traitement de l'asthme bronchique.

La présente invention a été réalisée à partir de la constatation qu'un groupe de dérivés de carbostyrile connus en tant qu'agents pour le traitement de l'asthme bronchique, comprennent des composés qui sont efficaces en tant qu'agents de traitement du glaucome, c'est-à-dire qu'ils sont aptes à abaisser la pression intraoculaire, ce qui est tout-à-fait inattendu et sans aucun rapport avec l'activité en tant qu'agent de traitement de l'asthme bronchique.

Les compositions pour le traitement du glaucome conformes à la présente invention, peuvent être mises sous forme de doses unitaires convenables en mélangeant un dérivé de formule (I) ou l'un de ses sels d'addition avec un

acide, avec un support usuel pour des préparations ophtalmiques. Ces compositions peuvent être mises sous forme de divers types usuels de doses unitaires, tels que des pommades ophtalmiques, des solutions ophtalmiques, etc..., pour l'administration locale,et des comprimés, granulés et solutions injectables pour l'administration par voie systémique. Il est particulièrement avantageux d'utiliser les compositions conformes à la présente invention sous forme de solutions ophtalmiques.

Bien que la dose d'administration des compositions de traitement conformes à la présente invention ne soit pas particulièrement limitée, celles-ci sont habituellement administrées à raison de 0,01 à 0,5 mg, et de préférence 0,05 à 0,1 mg par jour pour un adulte, la dose étant calculée en poids de constituant actif présent dans les compositions. Les compositions sont administrées, de préférence, en deux ou trois prises quotidiennes. Il est généralement avantageux que la teneur en constituant actif des compositions conformes à la présente invention, soit comprise entre 0,04 et 2 % en poids environ.

Les compositions de traitement conformes à la présente invention peuvent être préparées de façon classique à partir du dérivé de formule (I) ou de l'un de ses sels d'addition avec un acide en tant que constituant actif, en mélangeant celui-ci avec un excipient ou un support convenable et en mettant, si besoin est, le mélange sous la forme de dosage unitaire recherchée. Lorsqu'on prépare des pommades ou des solutions ophtalmiques ou des solutions injectables, on soumet ensuite ces compositions à une stérilisation. Les supports ou les diluants convenables sont choisis en fonction de la forme des compositions finales. Pour préparer des pommades ophtalmiques, on peut utiliser, par exemple, des supports émulsifiables, des supports solubles dans l'eau ou des supports pouvant être mis en suspension, tels que, en particulier, la vaseline blanche, la lanoline purifiée, la paraffine liquide, etc... Les solutions ophtalmiques sont préparées, en particulier, en utilisant de l'eau distillée stérile comme solvant.

Des agents de solubilisation, des stabilisants, des tampons, des antioxydants, des conservateurs peuvent, de plus, être incorporés dans les compositions conformes à la présente invention.Comme agents de solubilisation efficaces, on peut utiliser, par exemple, de la carboxyméthylcellulose de sodium ; des éthers de polyoxyéthylène tels que le lauryléther ou l'oléyléther de polyoxyéthylène ; des esters d'acide gras supérieurs et de polyéthylèneglycol tels que le monolaurate et le monooléate de polyéthylèneglycol ; des esters d'acides gras et de polyoxyéthylène tels que le monolaurate ou le monooléate de polyoxyéthylène sorbitan, etc... Comme stabilisants, on peut utiliser, par exemple, l'hydroxypropylméthylcellulose, l'alcool polyvinylique, la carboxyméthylcellulose, l'hydroxyéthylcellulose, la glycérine, l'EDTA, etc... Comme tampons utilisables dans le cadre de la présente invention, on peut citer, par exemple, le phosphate acide de sodium ou de potassium, l'acide borique, le borate de sodium, l'acide citrique, le citrate de sodium, l'acide tartrique, le tartrate de sodium, etc... Parmi les antioxydants efficaces, on peut citer, par exemple, le bisulfite de sodium, le thiosulfite de sodium, l'acide ascorbique, etc... On peut utiliser comme conservateurs, par exemple, le chlorobutanol, le chlorure de benzalkonium, le chlorure de cétylpyridinium, un sel de phénylmercure, le thimérosal, l'alcool phénéthylique, le méthylparabène, le propylparabène, etc...

Lorsqu'elles sont mises sous forme de solutions ophtalmiques, les compositions conformes à la présente invention doivent, de préférence, être isotoniques avec les larmes. Dans ce but, on peut éventuellement ajouter du sel ordinaire ou analogue, aux compositions. Il est souhaitable d'ajuster le pH des solutions ophtalmiques à une valeur comprise entre 5,5 et 8,5, et de préférence 6,5 et 7,5.

Les compositions pour le traitement du glaucome

conformes à la présente invention, préparées de la sorte, sont administrées aux malades en utilisant diverses méthodes adaptées à la forme des préparations. Les solutions ophtalmiques sont versées goutte-à-goutte dans l'oeil à l'aide d'un récipient approprié ou pulvérisées sur l'oeil à l'aide d'un applicateur. Les pommades ophtalmiques sont également appliquées sur l'oeil. Les comprimés et granules sont administrés par voie orale, tandis que les solutions injectables sont administrées par voie sous-cutanée, intramusculaire ou intraveineuse. On obtient l'effet thérapeutique recherché dans tous les cas.

Outre les dispositions qui précèdent, l'invention comprend encore d'autres dispositions, qui ressortiront de la description qui va suivre.

L'invention sera mieux comprise à l'aide du complément de description qui va suivre, qui se réfère à des exemples de préparation de la composition objet de la présente invention.

Il doit être bien entendu, toutefois, que ces exemples de préparation sont donnés uniquement à titre d'illustration de l'objet de l'invention, dont ils ne constituent en aucune manière une limitation.

<u>EXEMPLE 1 DE PREPARATION</u>

| | | |
|---|---|---|
| Chlorhydrate de 8-hydroxy-5-(1-hydroxy-2-isopropyl-aminobutyl) carbostyrile | | 0,2 g |
| Chlorure de benzalkonium | | 0,01 g |
| Phosphate monosodique | | 0,56 g |
| Phosphate monopotassique | | 0,8 g |
| Eau distillée | q.s.p. | 100 ml |

Les constituants sont dissous dans l'eau distillée, puis la solution est stérilisée par filtration sur un papier-filtre approprié. On obtient une composition de traitement du glaucome conforme à la présente invention, sous forme de solutions ophtalmiques.

EXEMPLE 2 DE PREPARATION

| | | |
|---|---|---|
| Chlorhydrate de 8-hydroxy-5-(1-hydroxy-2-isopropyl-aminobutyl)-3,4-dihydrocarbostyrile | | 0,2 g |
| Chlorure de benzalkonium | | 0,01 g |
| Phosphate monosodique | | 0,56 g |
| Phosphate monopotassique | | 0,8 g |
| Eau distillée | q.s.p. | 100 ml |

Les constituants sont dissous dans l'eau distillée et la solution est stérilisée par filtration sur du papier-filtre approprié. On obtient une composition de traitement du glaucome conforme à l'invention, sous forme de solution ophtalmique.

TEST D'EFFICACITE

Une goutte (environ 25 µl) de la solution ophtalmique préparée conformément à l'Exemple 1, est administrée deux fois par jour, matin et soir, pendant trois jours, dans chaque oeil, à trois hommes atteints d'un glaucome à angle ouvert. La pression intraoculaire de l'oeil est mesurée entre 10 et 11 heures du matin à l'aide d'un tonomètre à applanation Goldmann. Le Tableau ci-dessous rassemble les résultats obtenus.

TABLEAU

| Patient | | Pression intraoculaire (mm Hg) | | | |
|---|---|---|---|---|---|
| | | Avant l'administration | 1 jour après | 2 jours après | 3 jours après |
| A | O.G. * | 31,64 | 21,35 | 13,46 | 18,58 |
| | O.D. ** | 25,32 | 17,42 | 14,75 | 15,67 |
| B | O.G. | 38,85 | 23,78 | 24,52 | 21,27 |
| | O.D. | 35,43 | 21,47 | 23,38 | 20,69 |
| C | O.G. | 23,57 | 18,68 | 14,75 | 16,37 |
| | O.D. | 26,17 | 17,95 | 15,74 | 14,65 |

* oeil gauche

** oeil droit

Ce Tableau montre que la composition de traitement du glaucome conforme à la présente invention abaisse fortement la pression intraoculaire des malades atteints de glaucome et produit un effet thérapeutique remarquable.

L'efficacité de la composition de traitement du glaucome préparée conformément à l'Exemple 2 selon l'invention, est testée de la même façon. Elle fournit sensiblement le même abaissement de la pression intraoculaire.

Ainsi que cela ressort de ce qui précède, l'invention ne se limite nullement à ceux de ses modes de réalisation et d'application qui viennent d'être décrits de façon plus explicite ; elle en embrasse au contraire, toutes les variantes qui peuvent venir à l'esprit du technicien en la matière, sans s'écarter du cadre, ni de la portée, de la présente invention.

## REVENDICATION

Composition de traitement du glaucome, caractérisée en ce qu'elle comprend en tant que constituant actif, un dérivé de carbostyrile représenté par la formule (I) ci-après :

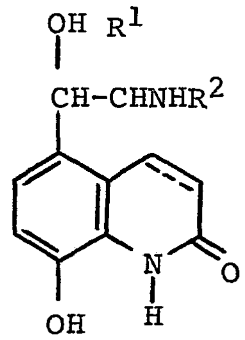

(I)

dans laquelle :

$R^1$ et $R^2$ représentent chacun un groupe alkyle inférieur, et

la liaison carbone-carbone entre les positions 3 et 4 du squelette carbostyrilique est une simple liaison ou une double-liaison,

ou l'un des sels d'addition avec un acide d'un tel dérivé.

# INTERNATIONAL SEARCH REPORT

International Application No PCT/JP79/00185

0020765

**I. CLASSIFICATION OF SUBJECT MATTER** (if several classification symbols apply, indicate all) [3]

According to International Patent Classification (IPC) or to both National Classification and IPC

A61K 31/47

**II. FIELDS SEARCHED**

Minimum Documentation Searched [4]

| Classification System | Classification Symbols |
| --- | --- |
| IPC | A61K 31/47 |
| IPC | C07D 215/22 |
| IPC | C07D 215/26 |

Documentation Searched other than Minimum Documentation to the Extent that such Documents are Included in the Fields Searched [5]

Osaka-fu Byoin Yakuzaishi-kai Henshu, Iyakuhin Yoran (Sogo Shinpan) Third Edition

**III. DOCUMENTS CONSIDERED TO BE RELEVANT** [14]

| Category* | Citation of Document,[16] with indication, where appropriate, of the relevant passages [17] | Relevant to Claim No.[18] |
| --- | --- | --- |
| A | JP, B2, 53-10994, 1978-4-18 | (1) |
| A | Osaka-fu Byoin Yakuzaishi-kai Henshu "Iyakuhin Yoran (Sogo Shinpan)" Third Edition (1976-8-20) Yakugyo Jihosha P.301 Ryokunaisho Chiryozai no Ko | (1) |

* Special categories of cited documents: [15]

"A" document defining the general state of the art

"E" earlier document but published on or after the international filing date

"L" document cited for special reason other than those referred to in the other categories

"O" document referring to an oral disclosure, use, exhibition or other means

"P" document published prior to the international filing date but on or after the priority date claimed

"T" later document published on or after the international filing date or priority date and not in conflict with the application, but cited to understand the principle or theory underlying the invention

"X" document of particular relevance

**IV. CERTIFICATION**

| Date of the Actual Completion of the International Search [2] | Date of Mailing of this International Search Report [2] |
| --- | --- |
| September 25, 1979 (25.09.79) | October 8, 1979 (08.10.79) |
| International Searching Authority [1] | Signature of Authorized Officer [20] |
| Japanese Patent Office | |